# EUROPEAN PATENT APPLICATION

(11) **EP 1 114 869 A1**
(43) Date of publication of application: **11.07.2001**
(21) Application number: 99943287.5
(22) Date of filing: 13.09.1999
(51) Int. Cl.: C12P 33/00

(54) **PROCESS FOR PRODUCING URSODEOXYCHOLATE**

(30) Priority: 14.09.1998 JP 25906898
(71) Applicant: KANSAI PAINT CO., LTD., Amagasaki-shi, Hyogo-ken 661-8555 (JP)
(72) Inventor: ODA, Shinobu, Hiratsuka-shi, Kanagawa 254-0016 (JP)
(74) Representative: Albrecht, Thomas, Dr.
(86) International application number: JP9904960
(87) International publication number: WO0015831

(57) **Abstract**

Disclosed is a process for producing an ursodeoxycholic acid ester in high yield which comprises the steps of depositing and immobilizing an anaerobic enteric bacterium capable of β-hydroxylating the keto group at the 7-position of bile acids and their esters (i.e., capable of producing 7β-hydroxysteroid dehydrogenase), on the surface of a hydrophilic immobilizing carrier containing nutrients and water therein and enclosed in an interface bioreactor; growing the bacterium to form a microbial film; bringing the microbial film into contact with a hydrophobic organic solvent having a 7-ketolithocholic acid ester dissolved therein; and cultivating the microbial film so as to reduce the 7-ketolithocholic acid ester microbiologically.

## Description

### Technical Field

### Background Art

This invention relates to a process for producing ursodeoxycholic acid esters by the utilization of an interface bioreactor. More specifically, this invention relates to a process for producing ursodeoxycholic acid esters which is carried out in such a way that the gaseous phase and the reaction solvent phase within the interface bioreactor are kept in an anaerobic condition.

In recent years, research and development concerning bioconversion involving the application of enzymes or microbial cells to organic synthesis have become active all over the world [Hiromichi Ohta, Yukigoseikagaku Kyokaishi, Vol. 41, 1018 (1983); Masashi Shimizu and Hideaki Yamada, Yukigoseikagaku Kyokaishi, Vol. 41, 1064 (1983)]. Among others, biocatalysts generally have high regio-selectivity and stereoselectivity, and are hence being applied to the synthesis of optically active substances such as raw materials for the manufacture of drugs, agricultural chemicals and liquid crystals. In particular, microbial conversion processes using grown microbial cells as a biocatalyst have several advantages, for example, in that they involve a low catalyst and in that they permit coenzyme-requiring reactions (e.g., oxidation and reduction reactions) to proceed smoothly.

One specific example of the application of microbial conversion to organic syntheses is the synthesis of steroid drugs. For example, the synthesis of androstadienes useful as raw materials for oral contraceptive pills [Konda, Hakko to Kogyo, Vol. 35, 861 (1977); Japanese Patent Laid-Open No. 59395/'79; Japanese Patent Laid-Open No. 67098/'79] and the synthesis of raw materials for steroid drugs such as adrenocortical hormones [Konda, Yukigoseikagaku Kyokaishi, Vol. 41, 1008 (1983)] have been reported. These processes have already been practiced on an industrial scale.

Meanwhile, a large number of microbial conversion processes for producing ursodeoxycholic acid that is useful for the dissolution of cholesteric gallstones have been reported. For example, the β-hydroxylation of the methylene group at the 7-position of lithocholic acid with the aid of a mold [H. Sawada et al., Appl. Environ. Microbiol., Vol. 44, 1249 (1982); T. Nihira et al., Appl. Environ. Microbiol., Vol. 54, 670 (1988); Japanese Patent Publication No. 3710/'89; Japanese Patent Publication No. 54478/'87; Japanese Patent Publication No. 29397/'91 (= U.S. Patent No. 4,579,819)] and the β-hydroxylation of the keto group at the 7-position of 7-ketolithocholic acid with the aid of an enteric bacterium [I.A. Macdonald et al., Appl. Environ. Microbiol., Vol. 44, 1187 (1982)] have been reported.

However, steroids including the aforesaid bile acids have a strong surface-active effect, i.e., the effect of destroying the cell membrane of microbial cells. In the case of reaction in a conventional aqueous system, therefore, the addition of an extremely low concentration of such a steroid to a culture of microbial cells causes the microorganism to die. Consequently, its microbial conversion is very difficult. For example, the maximum concentration of ursodeoxycholic acid accumulated as a result of the 7β-hydroxylation of lithocholic acid with the aid of Fusarium equiseti is as low as 1.2 g/l, even if the conversion conditions are optimized [S. Kulprrecha et al., Appl. Environ. Microbiol., Vol. 49, 338 (1985)].

Accordingly, it is the existing state of the art that ursodeoxycholic acid is being produced by an organic synthesis comprising multi-stage reactions, though this process has many disadvantages such as a low yield, the formation of by-products, the use of an alkali metal involving a safety problem, and a high production cost [Japanese Patent Publication No. 10062/'78; Japanese Patent Publication No. 54439/'87; Japanese Patent Publication No. 54720/'88; Japanese Patent Laid-Open No. 32692/'93].

The present inventor have previously proposed an interface bioreactor permitting the microbial conversion reaction of a high concentration of a hydrophobic toxic substrate [Japanese Patent No. 2542766; Oda and Ohta, Shikizai Kyokaishi, No. 70, 538 (1997)]. The interface bioreactor is a system in which a microorganism is grown in the form of a film at the solid/liquid interface between a hydrophilic immobilizing carrier (containing nutrients and water) and a hydrophobic organic solvent exhibiting no toxicity to the microorganism, and this microbial film is used as a biocatalyst to effect the microbial conversion of a lipophilic substrate added to the hydrophobic organic solvent. The interface bioreactor makes it possible to avoid the toxicity of a lipophilic substrate added to the reaction solvent in a substantial degree [S. Oda and H. Ohta, Biosci. Biotech. Biochem., Vol. 56, 1515 (1992)]. For example, where the interface bioreactor is used to estertfy cholesterol, various microorganism can grow actively even if cholesterol is added to the reaction solvent (oleyl alcohol) at a concentration of 12%. Consequently, the synthesis of cholesteryl octanoate by this esterification can proceed efficiently [S. Oda and H. Ohta, Biosci. Biotech. Biochem., Vol. 56, 2041 (1992)].

As the reaction solvent for the interface bioreactor, an aliphatic hydrocarbon such as decane is most commonly used. Such organic solvents generally have an oxygen dissolving capacity which is about ten time as high as that of water, and hence accelerates the growth of aerobic microorganisms and microbial oxidation reactions to proceed very efficiently [S. Oda et al., J. Ferment. Bioeng., Vol. 78, 149 (1994); J. Ferment. Bioeng., Vol. 80, 559 (1995); Biosci. Biotech. Biochem., Vol. 60, 83 (1996)].

On the other hand, the interface bioreactor is also applicable to microbial reduction [S. Oda and H. Ohta, Biosci. Biotech. Biochem., Vol. 56, 2041 (1992); T. Sugai et al., Tetrahedron, Vol. 51, 11987 (1995); S. Oda et al., Biosci. Biotech. Biochem., Vol. 62, 1762 (1998)]. From the viewpoint of enzyme reaction equilibrium, it is generally preferable to carry out such the reduction reaction in an anaerobic condition, because this is effective in enhancing the reaction rate.

The present inventor made intensive investigations with a view to developing an efficient process for producing an ursodeoxycholic acid ester in high yield. As a result, it has now been found that, when an anaerobic microorganism capable of β-hydroxylating the keto group at the 7-position of bile acids (more specifically, capable of producing 7β-hydroxysteroid dehydrogenase that is a stereoselective reductase) is used as a biocatalyst in an interface bioreactor, an ursodeoxycholic acid ester can be very efficiently produced by β-hydroxylating the keto group at the 7-position of a 7-ketolithocholic acid ester. The present invention has been completed on the basis of this finding.

### Disclosure of the Invention

Thus, the present invention provides a process for producing an ursodeoxycholic acid ester (hereinafter abbreviated as "UDCA ester") which comprises the steps of depositing and immobilizing an anaerobic enteric bacterium (hereinafter also referred to briefly as a "microorganism") capable of β-hydroxylating the keto group at the 7-position of bile acids and their esters, i.e., capable of producing 7β-hydroxysteroid dehydrogenase (hereinafter abbreviated as "7β-HSDH"), on the surface of a hydrophilic immobilizing carrier containing nutrients and water therein and enclosed in an interface bioreactor; growing the bacterium to form a microbial film; bringing the microbial film into contact with a hydrophobic organic solvent having a 7-ketolithocholic acid ester (hereinafter abbreviated as "7-KLCA ester")dissolved therein; and cultivating the microbial film so as to reduce the 7-KLCA ester microbiologically.

According to a preferred embodiment of the present invention, the above-described production of an UDCA ester is carried out after the interface bioreactor is brought into an anaerobic condition. That is, an UDCA ester can be very efficiently produced in high yield by carrying out the process of the present invention in an anaerobic condition after removing any oxygen from the gaseous phase within the interface bioreactor and, preferably, after further removing any oxygen dissolved in the hydrophobic organic solvent containing the substrate. Moreover, where a strictly anaerobic bacterium is used as the microorganism, an inert gas such as nitrogen gas is preferably introduced into the bioreactor during the reaction in order to secure a strictly anaerobic condition.

### Mode for Carrying Out the Invention

The anaerobic enteric bacteria capable of producing 7β-HSDH which can be used in the present invention may be enteric bacteria belonging to any desired genus, provided that they have the ability to produce 7β-HSDH. Specific examples thereof include enteric bacteria of the genus Clostridium, such as Clostridium absonum; enteric bacteria of the genus Eubacterium, such as Eubacterium aerofaciens; and enteric bacteria of the genus Peptostreptococcus, such as Peptostreptococcus productus. However, it is to be noted that Clostridium absonum produces 7α-HSDH in addition to 7β-HSDH. Since the Km value for 7α-HSDH is higher than that for 7β-HSDH, the formation of a chenodeoxycholic acid ester (hereinafter abbreviated as "CDCA ester") due to a reverse reaction (reduction) induced by 7α-HSDH becomes more predominant as the concentration of the 7-KLCA ester added is increased. Accordingly, where Clostridium absonum is used, it is desirable to reduce the concentration of the 7-KLCA ester dissolved in the organic solvent. On the other hand, Eubacterium aerofaciens and Peptostreptococcus productus do not pose the above-described problem because produce 7β-HSDH alone. Accordingly, when used in an anaerobic interface bioreactor, they can produce only an UDCA ester in high yield. Furthermore, since the 7β-HSDH of Clostridium absonum is an inducible enzyme [I.A. Macdonald and P.D. Roach, Biochim. Biophys. Acta, Vol. 665, 262 (1981); I.A. Macdonald et al., J. Lipid Res., Vol. 24, 1119 (1983)], it is necessary to add a slight amount of a substrate in advance and thereby effect enzyme induction during preliminary cultivation. On the other hand, the 7β-HSDH of Eubacterium aerofaciens and Peptostreptococcus productus is a constitutive enzyme [I.A. Macdonald et al., Appl. Environ. Microbiol., Vol. 44, 1187 (1982); S. Hirano and N. Masuda, Appl. Environ. Microbiol., Vol. 43, 1057 (1982)], no consideration for enzyme induction is required.

The deposition and immobilization of these microorganisms on the surfaces of a hydrophilic carrier may be carried out according to any of the per se known methods including, for example, the method described in Japanese Patent No. 2542766.

No particular limitation is placed on the material of the immobilizing carriers used in the present invention, provided that it has hydrophilic properties. There may be used any material that, when it is impregnated with, or brought into contact with, an aqueous solution containing nutrients, can supply the aqueous solution to a microorganism existing at its interface with an organic solvent. Specific examples thereof include natural high polymers such as alginic acid, carrageenan, starch matrix, agar and cellulosic materials (e.g., filter paper); synthetic high polymers such as polyvinyl alcohol, urethane polymers, polyacrylamide and polyacrylic acid; and inorganic materials such as foam glass and silica gel.

No particular limitation is placed on the form of these immobilizing carriers. They may be made in any of various forms such as fibers, film and granules. Alternatively, they may also be made in the form of cloth, nonwoven fabrics, paper, cardboards and the like.

In order to deposit and immobilize the aforesaid microorganism on these carriers, microbial cells are first deposited on the carriers, for example, by applying a suspension of microbial cells to, or spraying it on, the carriers which have previously been impregnated with an aqueous medium containing nutrients, by soaking the carriers in a culture of microbial cells, or by attaching microbial cells to the carriers by a suitable means and then supplying the carriers with an aqueous medium containing nutrients. Although the resulting carriers may preliminarily be cultivated in an aqueous medium containing nutrients, they are usually cultivated in contact with an organic solvent containing or not containing an organic compound used as a substrate, so as to grow the deposited microbial cells at the interface between the carrier and the organic solvent and thereby form an immobilized microbial film on the carriers. This cultivation causes the microorganism to become strongly attached to the carrier surfaces, so that the immobilized microbial film seldom separates from the carrier surfaces.

The nutrients used for the above-described cultivation of microbial cells may be chosen so as to be most suitable for the type of the microorganism used. They may comprise common nutrients including, for example, carbon sources such as glucose; nitrogen sources such as urea; minor metallic salts such as magnesium sulfate; and minor nutrients such as yeast extract and vitamins. Moreover, a satisfactory conversion activity can be achieved by the addition of a reducing agent (e.g., cysteine) which is peculiar to the cultivation of anaerobic microorganisms.

The cultivation may generally be carried out in a cultivation apparatus such as a thermostatic chamber or incubator. Alternatively, the cultivation may be carried out in a temperature-controlled reaction vessel where the carriers are immersed in an organic solvent containing or not containing a substrate and where an aqueous medium containing nutrients is also added thereto as required. The cultivation conditions such as incubation temperature and incubation time may be chosen so as to be suited for the type of the microorganism used.

In order to form a microbial film by the growth of microbial cells, it is usually preferable to carry out precultivation for one or more days. The microbial cells formed into the film on the surfaces of the hydrophilic carriers within the interface bioreactor as a result of the precultivation have much higher resistance to toxic substrates than free microbial cells, and hence permits the addition of a substrate at a high concentration and the accumulation of the resulting product. It is preferable to keep the microorganism-inoculated carriers in an anaerobic condition during the preliminary cultivation. To this end, it is desirable to deaerate the internal space of the vessel (usually the main body of the bioreactor) having the carriers placed therein and replace its atmosphere with nitrogen. However, if the precultivation time is unduly long, it shows a tendency to reduce productivity and enzyme activity. Usually, the best results can be achieved by carrying out the precultivation for approximately one day.

The 7-KLCA esters which can be used as substrates include, for example, alkyl esters (in particular, alkyl esters having an alkyl group of 1 to 6 carbon atoms) such as methyl and ethyl esters. Such 7-KLCA esters may be commercially available, or may be obtained in high yield and in highly pure form by esterifying 7-KLCA that can be easily prepared by the oxidation of CDCA with N-bromosuccinic acid. This esterification may be easily carried out according to a conventional process which comprises reacting 7-KLCA with a 1-alkanol (e.g., methanol or ethanol) in the presence of a mineral acid catalyst. In this step, the oil solubility of the resulting 7-KLCA ester can be enhanced by increasing the chain length of the ester group like butyl and hexyl groups. Consequently, its amount added to the reaction solvent of the anaerobic interface bioreactor can be increased.

As the hydrophobic organic solvent for dissolving the 7-KLCA ester, decane that is the commonest reaction solvent for use in interface bioreactors may be used for 7-KLCA esters having a long-chain ester group. However, it is usually preferable from the viewpoint of high-concentration production to use a hydrophobic organic solvent selected from among long-chain aliphatic ethers having some degree of polarity and typified by dihexyl ether and the like; long-chain aliphatic alcohols typified by oleyl alcohol and the like; aromatic ethers typified by diphenyl ether and the like; and long-chain aliphatic esters typified by decyl acetate and the like. Moreover, an appropriate solvent mixture composed of such solvents may also be used with consideration for the development of toxicity to microorganisms, the solubilities of the substrate and the product, and the like.

No particular limitation is placed on the concentration of the 7-KLCA ester in the aforesaid organic solvent, and it may vary according to the type of the ester, the type of the microorganism used, the solubility of the ester in the organic solvent, and the like. However, it may generally be in the range of 0.1 to 5% by weight and preferably 1 to 3% by weight.

Using the immobilizing carrier having a microbial film formed thereon in the above-described manner, the microbial conversion reaction of the 7-KLCA ester to an UDCA ester can be carried out by cultivating the microbial film in contact with the organic solvent having the 7-KLCA ester dissolved therein. This cultivation may be carried out in a suitable cultivation apparatus such as a thermostatic chamber, incubator or cultivation pack. In this step, the cultivation conditions such as incubation temperature, medium pH and incubation time may be chosen so as to be most suitable for the type of the microorganism used. Since the enteric bacterium capable of producing 7β-HSDH which is used in the present invention is anaerobic, it is generally preferable to carry out the cultivation in an anaerobic condition by removing any oxygen present in the dead spaces within the bioreactor and, more desirably, any oxygen dissolved in the hydrophobic organic solvent and by replacing its atmosphere with an inert gas such as nitrogen.

On the basis of the toxicity-alleviation phenomenon provided by the interface bioreactor [O. Oda and H. Ohta, Biosci. Biotech. Biochem., Vol. 56, 1515 (1992)], the UDCA ester produced by microbial reduction of the 7-KLCA ester is accumulated in the reaction solvent to a very high concentration. Usually, the concentration of the accumulated UDCA ester reaches a level which is several tens of times to several hundreds of times higher than that achieved by the reaction in an aqueous system. However, from the viewpoint of separation and purification of the UDCA ester, it is preferable to continue the cultivation in the bioreactor until the substrate (i.e., the 7-KLCA ester) is completely consumed.

The UDCA ester which has been accumulated to a high concentration can be recovered according to common separation and purification techniques, such as extraction with a polar solvent, crystallization by the addition of a nonpolar solvent, and column chromatography. The recovered UDCA ester may be converted to ursodeoxycholic acid (UDCA), for example, by saponification under ordinary alkaline hydrolysis conditions. The UDCA thus obtained may be recovered in pure form, for example, by recrystallization from a solvent such as ethyl acetate.

### Examples

The present invention is more specifically explained with reference to the following examples. However, it is to be understood that the present invention is not limited to these examples.

### Example 1

Using commercially available ABCM medium (manufactured by Eiken Chemical Co., Ltd.), a nutrient agar plate (with a surface area of 38.5 cm² and a volume of 25 ml) was prepared in a Petri dish made of glass. Then, 300 µl of a suspension of Eubacterium aerofaciens ATCC 35085 (1 loop per ml of ABCM medium) was spread to its surface and dried. This agar plate was precultivated in an anaerobic pack for one day to form a microbial film. After precultivation, a 1 wt.% solution of 7-KLCA methyl ester in dihexyl ether was layered thereover, and the agar plate was subjected to a stationary culture in an anaerobic pack at 37°C for 5 days. After completion of the reaction, the product accumulated in the dihexyl ether was determined by gas chromatography (using a silicone OV-210 column). As a result, the concentrations of formed UDCA methyl ester and unconverted 7-KLCA methyl ester were 8.2 g/l and 1.6 g/l, respectively.

### Example 2

Using an anaerobic agar plate type interface bioreactor similar to that used in Example 1, the reduction of 0.5 wt.% 7-KLCA ethyl ester with the aid of Clostridium absonum ATCC 27555 was carried out in the same manner as in Example 1. After 5 days of reaction, the concentration of the product obtained in the dihexyl ether was determined by gas chromatography. As a result, the concentrations of UDCA ethyl ester and 7-KLCA ethyl ester were 0.8 g/l and 2.3 g/l, respectively. In addition, 2.0 g/l of CDCA ethyl ester was formed as a by-product.

### Comparative Example 1

The conversion reaction of 7-KLCA methyl ester with the aid of Eubacterium aerofaciens ATCC 35085 was carried out by using ABCM liquid medium. After any oxygen present in 100 ml of the liquid medium and a culture flask (having an internal volume of 500 ml and fitted with a butyl gum plug) was removed and replaced with nitrogen, 10 ml of a culture broth of the microorganism was anaerobically inoculated with a syringe. After the microorganism was precultivated at 37°C for one day while being shaken at 100 strokes per minute, a solution of 7-KLCA methyl ester in dihexyl ether was injected with a syringe so as to give a 7-KLCA methyl ester concentration of 0.5% by weight. The conversion reaction was carried out for 5 days under the same conditions as employed for the precultivation. After completion of the reaction, the product was extracted with ethyl acetate, concentrated, and diluted with ethyl acetate. Then, the concentration of the formed product was determined by gas chromatography. As a result, it was confirmed that the desired reduction reaction scarcely proceeded because the steroid had a powerful inhibitory effect on the microorganism in the liquid culture system.

### Example 3

A plate-like carrier-packed anaerobic interface bioreactor in which the carriers comprised polyvinyl alcohol-glutaraldehyde/alginic acid-Ca²⁺ composite crosslinked gel plates. After the carriers were completely cured and washed, the aqueous phase within the gel was replaced with twice concentrated ABCM medium. Then, a one-day culture broth of Eubacterium aerofaciens ATCC 35085 was spread over the surface thereof, followed by precultivation under anaerobic conditions for one day. Six such carriers were packed in a stainless steel tank having an internal volume of 3 liters so as to be arranged in a vertical position. Thus, there was constructed a plate-like carrier-packed interface bioreactor as described in a publication [S. Oda et al., J. Ferment. Bioeng., Vol. 86, 84 (1998)]. After the internal space of this bioreactor was deaerated and replaced with nitrogen, 600 ml of a 5 wt.% solution of 7-KLCA methyl ester in oleyl alcohol having undergone deaeration and replacement with nitrogen was injected thereinto. Then, reaction was carried out at a temperature of 37°C and a stirring speed of 500 rpm for 14 days with the introduction of nitrogen (into the gaseous phase at a rate of 50 ml/min.). The formation of UDCA methyl ester by a reduction was recognized from the first day after the start of the reaction. The product accumulated smoothly with the lapse of time and reached a concentration of 49 g/l on the 14th day after the start of the reaction.

According to the above-described process of the present invention, ursodeoxycholic acid esters, which are important intermediates for the synthesis of ursodeoxycholic acid that is a bile acid useful for the dissolution of cholesteric gallstones, can be produced in very high yield and in highly pure form by charging an interface bioreactor with a 7-ketolithocholic acid ester as a substrate and effecting its microbial reduction in an anaerobic condition with the aid of an anaerobic enteric bacterium.

## Claims

1. A process for producing an ursodeoxycholic acid ester which comprises the steps of depositing and immobilizing an anaerobic enteric bacterium capable of producing 7β-hydroxysteroid dehydrogenase, on the surface of a hydrophilic immobilizing carrier containing nutrients and water therein and enclosed in an interface bioreactor; growing the bacterium to form a microbial film; bringing the microbial film into contact with a hydrophobic organic solvent having a 7-ketolithocholic acid ester dissolved therein; and cultivating the microbial film so as to reduce the 7-ketolithocholic acid ester microbiologically.

2. A process as claimed in claim 1 wherein the process is carried out in an anaerobic condition after removing any oxygen present in the dead spaces within the bioreactor and any oxygen dissolved in the hydrophobic organic solvent.

3. A process as claimed in claim 1 wherein the anaerobic enteric bacterium is selected from the group consisting of Clostridium absonum, Eubacterium aerofaciens and Peptostreptococcus productus.

4. A process as claimed in claim 1 wherein the 7-ketolithocholic acid ester is an alkyl ester of 7-ketolithocholic acid.

5. A process as claimed in claim 1 wherein the hydrophobic organic solvent is a long-chain aliphatic ether, an aromatic ether, a long-chain aliphatic alcohol, a long-chain aliphatic ester, or a mixture of such organic solvents.

6. An ursodeoxycholic acid ester produced by the process of claim 1.
